# EUROPEAN PATENT APPLICATION

(11) **EP 4 094 764 A1**
(43) Date of publication of application: **30.11.2022**
(21) Application number: 21382471.7
(22) Date of filing: 24.05.2021
(51) Int. Cl.: A61K 31/4704, A61P 21/00

(54) **1,2-DIHYDROQUINOLINE-2-ONES FOR THEIR USE IN THE TREATMENT OF LIMB-GIRDLE MUSCULAR DYSTROPHY**

(71) Applicant: Consejo Superior De Investigaciones Científicas, 28006 Madrid (ES); Administración General de la Comunidad Autónoma de Euskadi, 01010 Vitoria-Gasteiz (ES)
(72) Inventor: MARTÍNEZ GIL, Ana, E-28006 Madrid (ES); PALOMO RUIZ, Valle, E-28006 Madrid (ES); SÁENZ PEÑA, Miren Ametsa, E-20014 San Sebastián (ES); LÓPEZ DE MUNAIN ARREGI, Adolfo José, E-20014 San Sebastián (ES)
(74) Representative: ABG Intellectual Property Law, S.L.

(57) **Abstract**

The present invention relates to 1,2-dihydroquinoline-2-ones of formula (I): 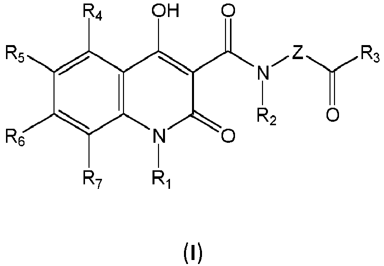 or a pharmaceutically acceptable salt or solvate thereof, for its use in the treatment of limb girdle muscular dystrophy.

## Description

### FIELD OF THE INVENTION

The present invention belongs to the field of medicinal chemistry. In particular, the invention relates to a method for the treatment of a particular type of muscular dystrophy, namely Limb-Girdle Muscular Dystrophy (LGMD), including limb girdle muscular dystrophy R1 calpain 3-related, also known as LGMDR1.

### BACKGROUND

Muscular dystrophy (MD) refers to a group of muscle diseases characterised by progressive skeletal muscle weakness, defects in muscle proteins, and the death of muscle cells and tissue. Different diseases including Duchenne, Becker, limb girdle, congenital, facioscapulohumeral, myotonic, oculopharyngeal, distal, and Emery-Dreifuss are always classified as MD but there are more than one hundred diseases in total with similarities to MD.

Most types of MD are multi-system disorders with manifestations in body systems including the heart, gastrointestinal and nervous systems, endocrine glands, skin, eyes and even brain. The condition may also lead to mood swings and learning difficulties. The symptoms usually begin during the first two decades of life and the disease gradually worsens, often resulting in loss of walking ability 10 or 20 years after onset. Subsequently, muscle degeneration progresses becoming a highly disabling disease that prevents patients from performing simple daily tasks.

In Datta et al. (Current Neurology and Neuroscience Reports, 2020, 20:14) an overview on muscular dystrophies is described. Historically, these disorders are considered to be difficult to treat. However, in the last three decades, many efforts have been made in the development of novel treatments and biomarkers in the realm of muscular dystrophies commonly encountered in pediatric population.

Among the muscular dystrophy diseases, Limb-Girdle Muscular Dystrophy (LGMD) was first proposed by Walton and Nattrass in 1954 as part of a classification of muscular dystrophies. LGMD is characterised by progressive symmetrical atrophy and weakness of the proximal limb muscles and by elevated serum creatine kinase. Muscle biopsies demonstrate dystrophic lesions and electromyograms show myopathic features.

Different LGDM therapeutic strategies have been proposed (Current Neurology and Neuroscience Reports, 2020, 20:14), such as the use of corticosteroids (prednisone, deflazacort), anti-oxidants (epicatechin cacao flavonoid and epigallocatechin gallate green tea polyohenol), anti-fibrotic agents (CoQ10 and lisinopril), anti-myostatin, gene-based therapies and antisense oligonucleotides. However, there is no standard of care medical therapy for LGMDs and no specific biomarkers for diagnosis other than genetic testing.

Several types of LGMD exist, including Limb-Girdle Muscular Dystrophy R1 calpain 3-related, also known as LGMDR1, LGMD2A or calpainopathy. This type is an autosomal recessive muscular dystrophy due to mutations in the CAPN3 gene that causes progressive degeneration of the proximal muscles of the pelvic and shoulder girdle.

The correct homeostasis between the synthesis and degradation of proteins in the muscle fibre is key to maintain the muscle and thus to avoid muscle atrophy and weakness (Scicchitano et al., 2018). For that purpose, there are certain signalling pathways, such as the Akt/mTOR pathway, which stimulates protein synthesis, myofibre growth and inhibits protein degradation (Glass, 2005). The ERK1/2 MAPK pathway, which controls the growth and differentiation of muscle cells, is also involved in myofibre maintenance in adults (Jones et al., 2001; Roux et al., 2004; Seaberg et al, 2015). Finally, among other pathways, the Wnt signalling participates in differentiation during muscle development and in the regeneration of muscle fibre in adults (von Maltzahn et al., 2012).

According to Jaka et al. (Expert Reviews in Molecular Medicine, 2017, 19, 1-16), two methods are used for activating the Wnt signalling pathway: genetic silencing of FRZB and administration of LiCl. Both approaches have produced an increase in β1D integrin levels that are downregulated in patients' myotubes.

The regulation of FRBZ expression is proposed as a potential therapeutic target given that *in vitro* studies support the idea that it may be possible to bring expression and phosphorylation of various proteins back towards appropriate levels in LGMDR1 patients. Specifically, silencing this gene has caused the β1D integrin to return to normal levels in myotubes from LGMD2A patients. Furthermore, since it was known that FRBZ regulates the localization of β-catenin down-stream of the Wnt pathway, it is proposed that FRZB may play a role in the crosstalk between integrin and Wnt-signalling pathway.

On the other hand, the treatment with LiCI, although with some differences, produced similar results to those obtained by siFRBZ, observing lower levels of expression of the FRBZ gene, as well as higher levels of the β1D adult isoform of the integrin in primary myotubes. Due to its activator role in the Wnt signalling pathway, certain studies showed beneficial *in vitro* results (Du et al 2009, Yang et al 2011, Abu-Baker et al 2013, Jaka et al 2017). However, the participation of lithium has only been shown in the Wnt signalling pathway, but not in other signalling pathways implicated in the development of LGMDR1. Furthermore, it is mentioned that the treatment with LiCI is associated with an inhibitory effect on GSK3β.

In fact, when the Wnt signaling pathway is active, the Wnt ligands induce the inactivation of GSK3β preventing β-catenin phosphorylation, allowing its accumulation in the cytoplasm and translocating it to the nucleus acting as a transcription factor. On the contrary, when the Wnt signaling pathway is inactive, GSK3β is activated. It phosphorylates β-catenin so that it is subsequently degraded (MacDonald et al., 2009).

Kurgan et al. (Cells, 2019, 8, 134) disclose that a low therapeutic dose of lithium (0.5 mM) has shown to inhibit GSK3 and to augment myoblast fusion, suggesting that non-toxic dose of lithium might be an effective option for promoting muscle development *in vivo.* However, no particular diseases are mentioned in this document, but some indications regarding the use of lithium for potentially attenuating some of the muscle atrophy observed in different conditions.

Whitley et al. (Physiological Reports, 2020, 8(14): e 14517) also report that low dose of LiCI treatment is effective in inhibiting GSK3β and enhances fatigue resistance in some muscles via NFAT activation.

On the other hand, the compound known as VP0.7 is a highly selective and allosteric modulator of GSK3 that reversibly inhibits this kinase (Luna-Medina et al., Journal of Neuroscience, 2007, 27(21), 5766-5776; Palomo et al., J. Medicinal Chemistry, 2011, 54(24), 8461-8470). This compound and a derivative thereof have been shown to be active in preclinical models of fragile X (Franklin et al., Biological Psychiatry, 2014, 75,198-206) and multiple sclerosis (Beurel et al., J. Immunology, 2013, 190:5000-11). VP0.7 has also been used, among many other GSK3 inhibitors, in formulations for facilitating or expanding the generation of tissue cells in a stem cell population (US2017/0252449, WO2018/191350 and WO2020/081838). A derivative of this compound has been reported to correct delayed myogenesis in myoblasts from patients with type I congenital myotonic dystrophy (CDM1) (Palomo et al., J. Medicinal Chemistry, 2017, 60(12), 4983-5001).

In view of all above, no effective therapies exist for the treatment of limb girdle muscular dystrophy, and more particularly limb girdle muscular dystrophy R1 calpain 3-related (LGMDR1). In fact, the pathophysiological mechanism by which the absence of calpain 3 provokes the atrophy in muscles is not clear. Thus, there is a great need to know the alterations in the expressions of proteins involved in signalling pathways, such as Wnt and mTOR, of LGMDR1 patient's muscles, and to look for new therapeutic agents to treat this disease.

### BRIEF DESRIPTION OF THE INVENTION

The authors of the present invention have found that some 1,2-dihydroquinoline-2-ones, such as VP0.7, restore the expression and phosphorylation of key proteins involved in signalling pathways that regulate muscle homeostasis, both Wnt and mTOR pathways, and which are significantly reduced in LGMDR1patients' muscles, thus opening the potential use of said compounds as a therapeutic option in the treatment of said disease.

Namely, the results obtained by the inventors have allowed to establish that there is a reduction of active β-catenin in the muscle of said patients confirming that Wnt pathway is altered. In addition to that, alteration of the mTOR signalling pathway has also been observed in muscle from LGMDR1patients due to the reduced amount of the mTOR protein and its phosphorylation.

By administering the compounds of the invention in myotubes of LGMDR1patients, a complete inhibition of GSK3β has been observed which led to an increase in the amount of β-catenin and to an activation of the mTOR pathway. The restoration of said pathways supports the potential benefit of said 1,2-dihydroquinoline-2-ones to be used as a therapeutic agents in the treatment for LGMDR1patients. Furthermore, some experiments have pointed out that the treatment with said compounds is not affecting other tissues avoiding undesired adverse effects.

Thus, the main aspect of the present invention refers to 1,2-dihydroquinoline-2-one compounds of formula (I): 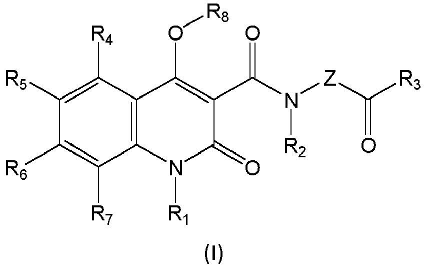 wherein:
R₁ is selected from H, an optionally substituted C₁-C₁₂ alkyl group and an optionally substituted C₂-C₅ alkenyl group;
R₂ is H or a C₁-C₆ alkyl group;
R₃ is an optionally substituted C₁-C₂₀ alkyl group;
R₄-R₇ are independently selected from H, halogen, an optionally substituted C₁-C₆ alkyl group and -OR₉, wherein R₉ is hydrogen or an optionally substituted C₁-C₆ alkyl;
R₈ is selected from H and an optionally C₁-C₆ alkyl group;
Z is selected from -NR₁₀- and an optionally substituted phenylene; wherein R₁₀ is selected from H and an optionally substituted C₁-C₄ alkyl;
or a pharmaceutically acceptable salt or solvate thereof,
for its use in the treatment of limb girdle muscular dystrophy.

The present invention also relates to the use of the above defined compounds of formula (I) in the manufacture of a medicament for the treatment of limb girdle muscular dystrophy.

Additionally, the invention is also directed to a method for the treatment of limb girdle muscular dystrophy comprising the administration of an effective amount of a compound of formula (I) as defined above, or a or a pharmaceutically acceptable salt or solvate thereof, to a patient in need thereof.

### BRIEF DESCRIPTION OF THE FIGURES

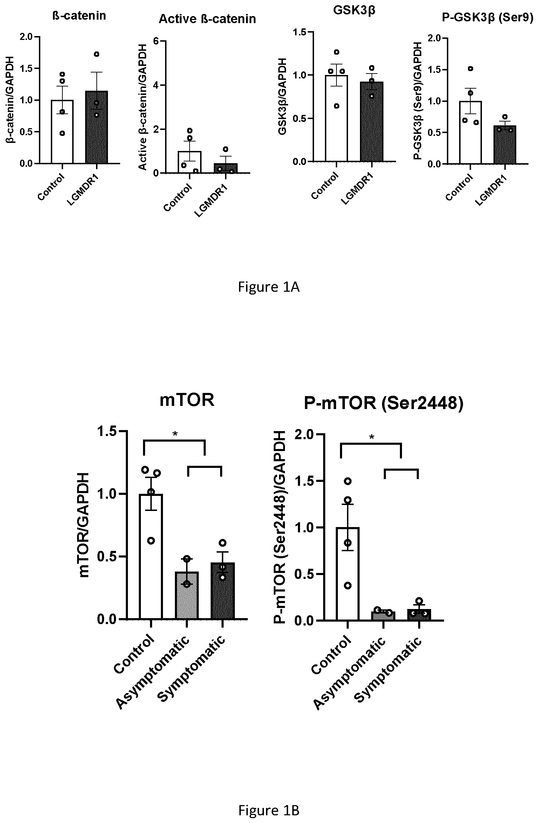

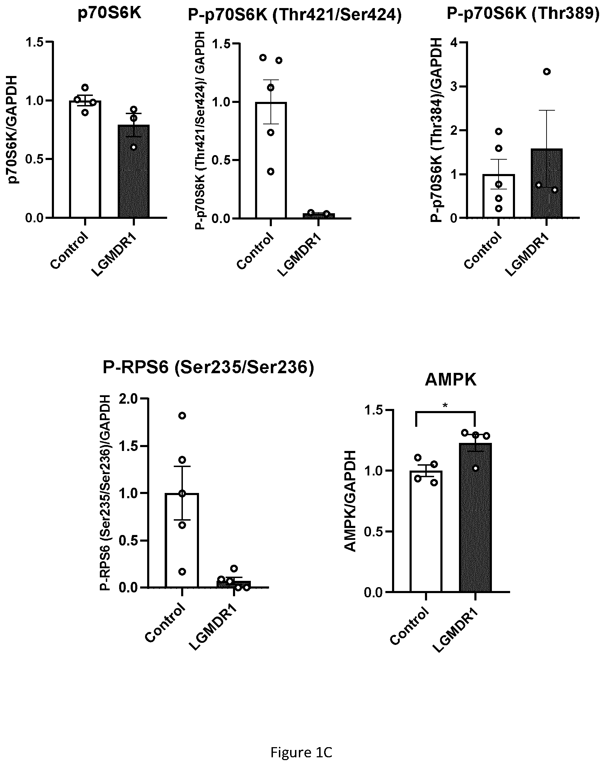

Figure 1. Quantification of Wnt and Akt/mTOR pathway proteins by Western blot in muscle from controls and LGMDR1 patients. A) Quantification of Wnt pathway proteins: β-catenin, active β-catenin, GSK3β and P-GSK3β (Ser9); B) Quantification of Akt/mTOR pathway proteins: mTOR and P-mTOR (Ser2448). C) Detection of p70S6K, P-p70S6K (Thr421/Ser424), P-p70S6K (Thr389), P-RPS6 (Ser235/Ser236) and AMPK. Loading control: GAPDH.

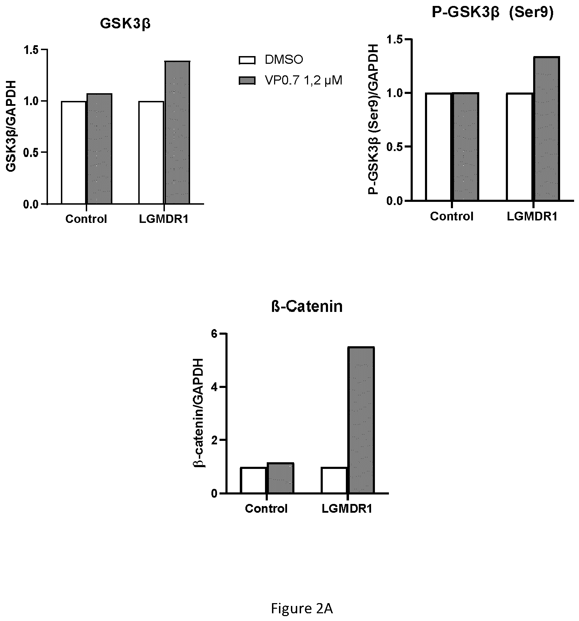

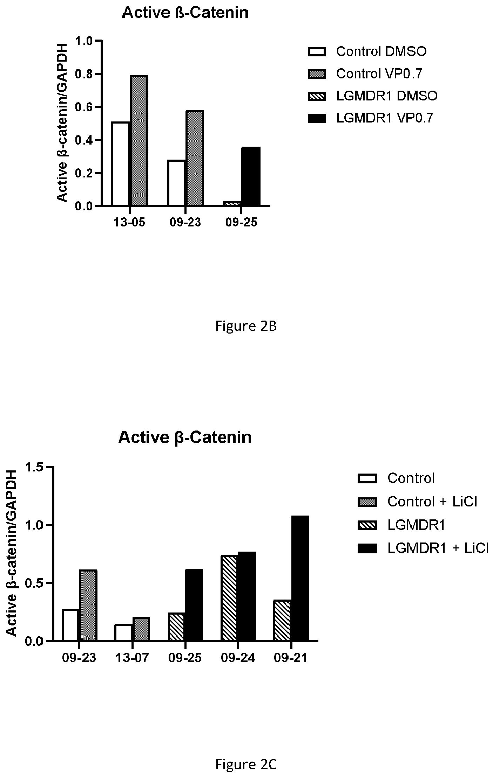

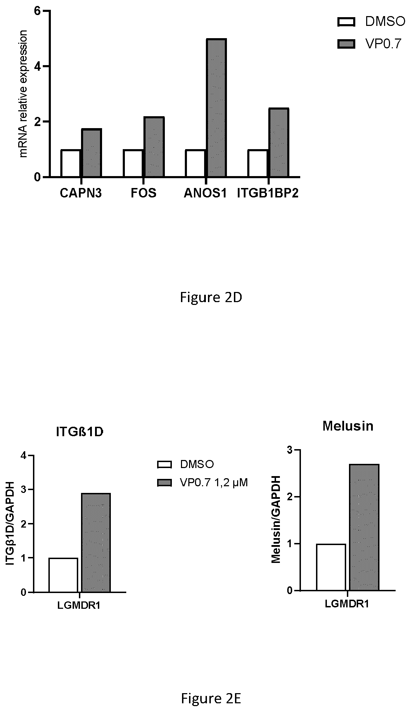

Figure 2. Activation of the Wnt pathway in human myotubes at day 8 of differentiation and after 48h of treatment with VP0.7 at a concentration of 1.2 µM in control and LGMDR1 patients. A) Western blot of GSK3β, P-GSK3β (Ser9) and total β-catenin. B) Western blot of active β-catenin; C) Western blot of active β-catenin after administration of LiCI; D) Expression of CAPN3, FOS, ANOS1 and ITGB1BP2 genes in LGMDR1 myotubes. E) Western blot of structural proteins integrin β1D and melusin.

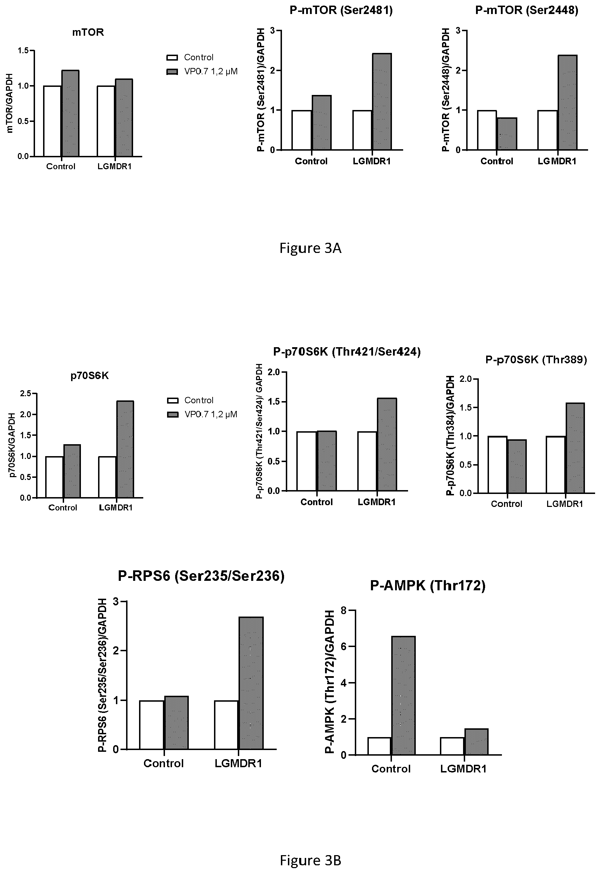

Figure 3. Activation of the Akt/mTOR pathway in human myotubes at day 8 of differentiation and after 48h of treatment with VP0.7 at a concentration of 1.2 µM in control and LGMDR1 patients. A) Western blot of mTOR and P-mTOR (Ser2448 and Ser2481); B) Western blot of p70S6K, P-p70S6K (Thr421/Ser424), P-p70S6K (Thr389), P-RPS6 (Ser235/Ser236), and P-AMPK (Thr 172).

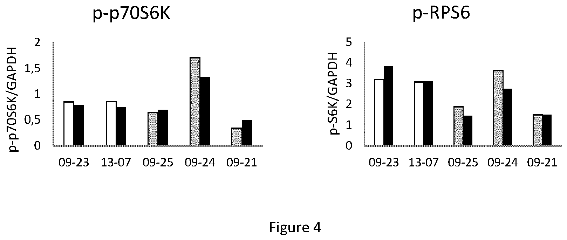

Figure 4. Analysis of the Akt/mTOR pathway in human myotubes after treatment with LiCI at a concentration of 10 mM in control and LGMDR1 patients. Densitometry results of the Western blot of the phosphorylated forms of p70S6K and RPS6 proteins. White: control samples without treatment. Grey: LGMDR1 samples without treatment. Black: LiCI treatment.

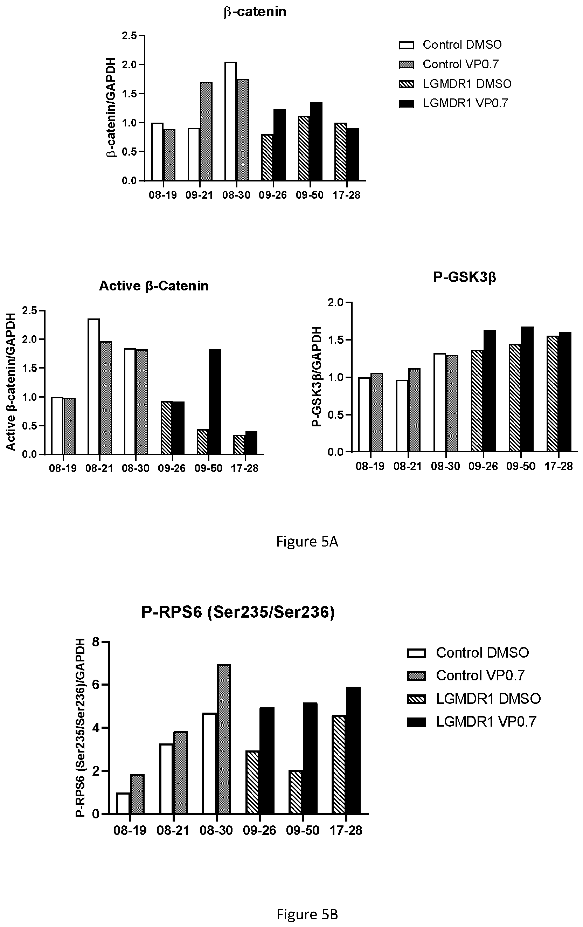

Figure 5. Analysis of the effect in protein expression on fibroblasts upon treatment with VP0.7. A) Western blot of β-catenin and its activated form. B) Western blot of p-GSK3β and P-RPS6.

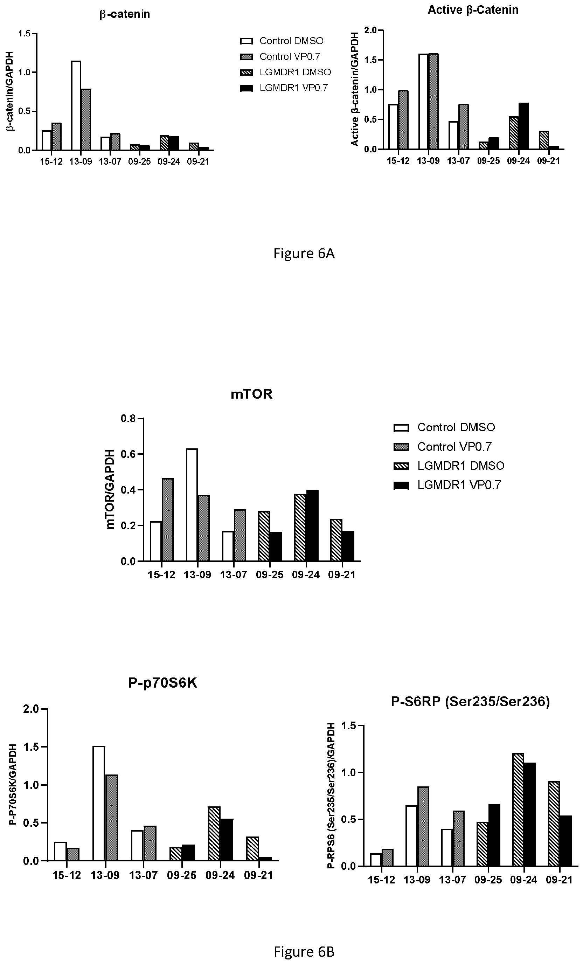

Figure 6. Analysis of the effect in protein expression on CD56-cells upon treatment with VP0.7. A) Western blot of β-catenin and its activated form. B) Western blot of mTOR, p70S6K and P-RPS6.

### DETAILED DESCRIPTION OF THE INVENTION

In the above definition of compounds of formula (I), the following terms have the meaning indicated:
"Alkyl" refers to a linear or branched hydrocarbon chain radical consisting of carbon and hydrogen atoms, containing no unsaturation, having one to twenty carbon atoms (C₁-C₂₀), or one to twelve carbon atoms (C₁-C₁₂), or one to six carbon atoms (C₁-C₆) or one to four carbon atoms (C₁-C₄), more preferably one to three carbon atoms (C₁-C₃ alkyl), even more preferably one to two carbon atoms (C₁-C₂ alkyl), and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halogen, hydroxy, alkoxy, carboxy, cyano, carbonyl, acyl, alkoxycarbonyl, amino, nitro, cycloalkyl and aryl.
"Alkenyl" refers to linear or branched hydrocarbon chain radical consisting of carbon and hydrogen atoms having at least one double bond, and from 2 to 6 carbon atoms, more preferably having 2, 3 or 4 carbon atoms. In a preferred embodiment, they refer to linear hydrocarbons having a single double bond, and from 2 to 6 carbon atoms, preferably 2 or 3 carbon atoms. Alkenyl radicals may be optionally substituted by one or more substituents such as halogen, hydroxy, alkoxy, carboxy, cyano, carbonyl, acyl, alkoxycarbonyl, amino and nitro.
"Aryl" refers to an aromatic group having between 6 to 18 carbon atoms, preferably between 6 and 12 carbon atoms, more preferably between 6 and 10 and even more preferable having 6 carbon atoms, comprising 1, 2 or 3 aromatic nuclei, including for example and in a non-limiting sense, phenyl, biphenyl, naphthyl, indenyl, phenanthryl, anthracyl or terphenyl. Preferably, aryl refers to phenyl (Ph) or naphthyl. The aryl radical may be optionally substituted by one or more substituents such as hydroxy, mercapto, halogen, alkyl, phenyl, alkoxy, haloalkyl, aralkyl, nitro, cyano, dialkylamino, aminoalkyl, acyl and alkoxycarbonyl, as defined herein. The aryl group can also be fused to another non-aromatic ring, such as a cycloalkyl or a heterocyclyl.
"Cycloalkyl" refers to a saturated or partially saturated mono- or bicyclic aliphatic group having between 3 and 10, preferably between 3 and 6 carbon atoms ("C₃-C₆ cycloalkyl"), which is bound to the rest of the molecule by means of a single bond, including for example and in a non-limiting sense, cyclopropyl, cyclohexyl or cyclopentyl. Unless otherwise stated specifically in the specification, the term "cycloalkyl" is meant to include cycloalkyl radicals which are optionally substituted by one or more such as alkyl, halo, hydroxy, amino, cyano, nitro, alkoxy, carboxy and alkoxycarbonyl.
"Halogen" refers to bromo, chloro, iodo or fluoro.
"Carboxy" refers to a radical of the formula -C(O)OH.
"Alkoxy" refers to a radical of the formula -ORₐ where Rₐ is an alkyl radical as defined above, e. g., methoxy, ethoxy, propoxy, etc.
"Alkoxycarbonyl" refers to a radical of the formula -C(O)ORₐ where Rₐ is an alkyl radical as defined above, e. g., methoxycarbonyl, ethoxycarbonyl, propoxycarbonyl, etc.
"Amino" refers to a radical of the formula-NH₂, -NHRₐ or -NRₐR_{b}, wherein Rₐ and R_{b} are alkyl radicals as defined above.
"Acyl" refers to a radical of the formula-C(O)-Rc and -C(O)-Rd, where Rc is an alkyl radical as defined above and Rd is an aryl radical as defined above, e. g., acetyl, propionyl, benzoyl, and the like.
"Phenylene" refers to a biradical -(C₆H₄)- which can be optionally substituted by one or more substituents such as hydroxy, mercapto, halogen, alkyl, alkoxy, nitro, cyano, dialkylamino, aminoalkyl, acyl and alkoxycarbonyl, as defined herein. The phenylene group can also be fused to another aromatic or non-aromatic ring, such as a cycloalkyl or a heterocyclyl.
The term "pharmaceutically acceptable salts or solvates" refers to any pharmaceutically acceptable salt or solvate which, upon administration to the recipient, is capable of providing (directly or indirectly) a compound as described herein. The preparation of salts and solvates can be carried out by methods known in the art.

For instance, pharmaceutically acceptable salts of compounds used in the present invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two.

The compounds used in the present invention may be in crystalline form either as free compounds or as solvates (e.g. hydrates). Methods of solvation are generally known within the art. In a particular embodiment the solvate is a hydrate.

The compounds of formula (I), or their salts or solvates, are preferably used in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts or solvates.

The compounds used in the present invention represented by the above described formula (I) may include enantiomers depending on the presence of chiral centres or isomers depending on the presence of multiple bonds (e.g. Z, E). The single isomers, enantiomers or diastereoisomers and mixtures thereof can be used in the present invention.

In a preferred embodiment, R₁ is H, a linear or branched C₁-C₆ alkyl group optionally substituted with an aryl or cycloalkyl group, or a non-substituted C₂-C₅ alkenyl group. More preferably R₁ is H, a non-substituted linear or branched C₁-C₆ alkyl, even more preferably R₁ is a non-substituted linear or branched C₁-C₄ alkyl.

Representative substituents that can be used as R₁ are methyl, ethyl, n-propyl, isopropyl, n-butyl, iso-butyl, tert-butyl, sec-butyl, n-hexyl, -CH₂-phenyl, -CH₂-cyclopropyl and isoprenyl. More preferably, R₁ is methyl, ethyl, n-propyl, isopropyl, n-butyl, iso-butyl, -CH₂-phenyl, -CH₂-cyclopropyl or isoprenyl. Even more preferably, R₁ is ethyl.

In another preferred embodiment, R₂ is H or a non-substituted C₁-C₃ alkyl group, more preferably is H.

In another preferred embodiment, R₃ is a non-substituted C₁-C₂₀ alkyl group, more preferably a non-substituted C₄-C₁₅ alkyl group, even more preferably a non-substituted C₄-C₁₂ alkyl group. Most preferably R₃ is a non-substituted C₁₁ alkyl group.

In another preferred embodiment, R₄, R₅, R₆ and R₇ are independently selected from hydrogen, halogen, substituted or unsubstituted C₁-C₆ alkyl and -OR₉, wherein R₉ is selected from hydrogen and substituted or unsubstituted C₁-C₆ alkyl.

More preferably, R₄, R₅, R₆, and R₇ are independently selected from H, non-substituted C₁-C₄ alkyl, halogen and hydroxy. Even more preferably R₄, R₅, R₆, and R₇ are independently selected from H and halogen.

Most preferred are compounds wherein R₄-R₇ are H.

In another preferred embodiment, Z is selected from -NR₁₀- and a non-substituted phenylene, wherein R₁₀ is H or a C₁-C₄ alkyl chain optionally substituted with phenyl. More preferably, Z is selected from -NH- and a non-substituted phenylene, even more preferably Z is -NH-.

In another preferred embodiment:
- R₁ is a non-substituted C₁-C₆ alkyl group;
- R₂ is H;
- R₃ is a non-substituted linear C₄-C₁₅ alkyl group;
- R₄-R₇ are independently selected from H and halogen;
- Z is selected from -NH- and non-substituted phenylene.

Representative compounds of formula (I) are the following: 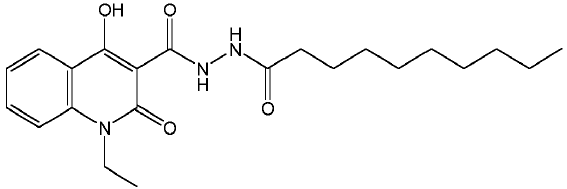 N'-decanoyl-1-ethyl-4-hydroxy-2-oxo-1,2-dihydroquinoline-3-carbohydrazide 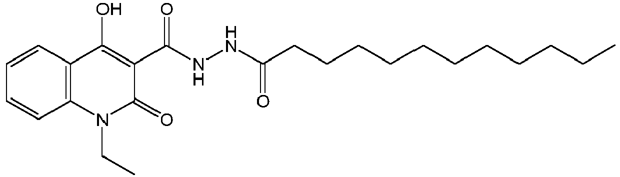 N'-dodecanoyl-1-ethyl-4-hydroxy-2-oxo-1,2-dihydroquinoline-3-carbohydrazide 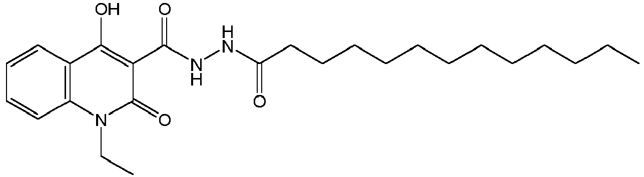 N'-tridecanoyl-1-ethyl-4-hydroxy-2-oxo-1,2-dihydroquinoline-3-carbohydrazide 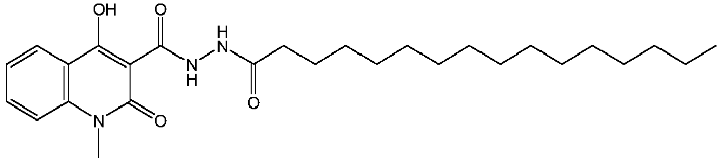 N'-hexadecanoyl-1-methyl-4-hydroxy-2-oxo-1,2-dihydroquinoline-3-carbohydrazide 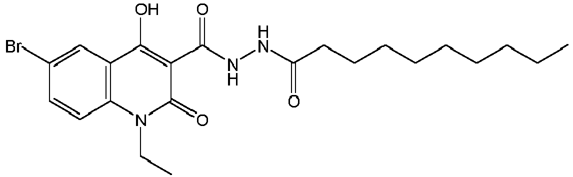 N'-decanoyl-6-bromo-1-ethyl-4-hydroxy-2-oxo-1,2-dihydro-quinoline-3-carbohydrazide 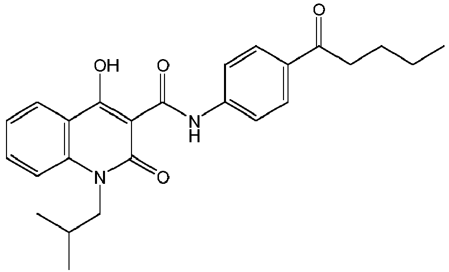 4-hydroxy-1-isobutyl-2-oxo-1,2-dihydro-quinoline-3-carboxylic acid (4-pentanoyl-phenyl)-amide 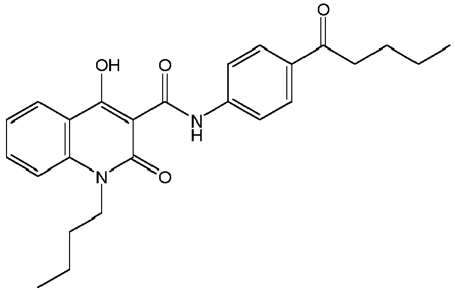 1-butyl-4-hydroxy-2-oxo-1,2-dihydro-quinoline-3-carboxylic acid (4-pentanoyl-phenyl)-amide 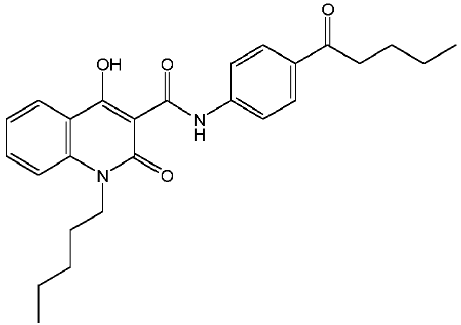 4-hydroxy-2-oxo-1-pentyl-1,2-dihydro-quinoline-3-carboxylic acid (4-pentanoyl-phenyl)-amide 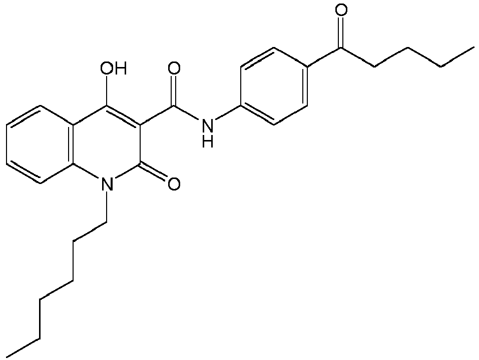 1-hexyl-4-hydroxy-2-oxo-1,2-dihydro-quinoline-3-carboxylic acid (4-pentanoyl-phenyl)-amide

The most preferred compound of formula (I) is:

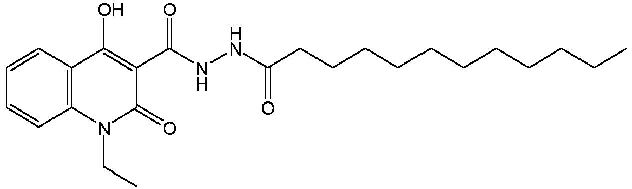

N'-dodecanoyl-1-ethyl-4-hydroxy-2-oxo-1,2-dihydroquinoline-3-carbohydrazide, also known as VP0.7.

The compounds of formula (I) defined above and used in the present invention can be obtained by available synthetic procedures. Some examples of these procedures are described in EP2769720.

The present invention also relates to the use of the above defined compounds of formula (I) in the manufacture of a medicament for the treatment of limb girdle muscular dystrophy.

Additionally, the invention is also directed to a method for the treatment of limb girdle muscular dystrophy comprising the administration of an effective amount of a compound of formula (I) as defined above, or a pharmaceutically acceptable salt or solvate thereof, to a patient in need thereof.

In a particular embodiment, the limb girdle muscular dystrophy includes both recessive limb girdle muscular dystrophies (LGMD R) and dominant limb girdle muscular dystrophies (LGMD D).

Within this particular embodiment, recessive limb girdle muscular dystrophies are selected from LGMD R1 calpain3-related (LGMDR1 or calpainopathy), LGMD R2 dysferlin-related (LGMDR2), LGMD R3 α-sarcoglycan-related (LGMDR3), LGMD R4 β-sarcoglycan-related (LGMDR4), LGMD R5 γ-sarcoglycan-related (LGMDR5), LGMD R6 δ-sarcoglycan-related (LGMDR6), LGMD R7 telethonin-related (LGMDR7), LGMD R8 TRIM 32-related (LGMDR8), LGMD R9 FKRP-related (LGMDR9), LGMD R10 titin-related (LGMDR10), LGMD R11 POMT1-related (LGMDR11), LGMD R12 anoctamin5-related (LGMDR12), LGMD R13 Fukutin-related (LGMDR13), LGMD R14 POMT2-related (LGMDR14), LGMD R15 POMGnT1-related (LGMDR15), LGMD R16 α-dystroglycan-related (LGMDR16), LGMD R17 plectin-related (LGMDR17), LGMD R18 TRAPPC11-related (LGMDR18), LGMD R19 GMPPB-related (LGMDR19), LGMD R20 ISPD-related (LGMDR20), LGMD R21 POGLUT1-related (LGMDR21), LGMD R22 collagen6-related (LGMDR22), LGMD R23 laminin α2-related (LGMDR23), LGMD R24 POMGNT2-related (LGMDR24).

Within said particular embodiment, dominant limb girdle muscular dystrophies are selected from LGMD D1 DNAJB6-related (LGMD D1), LGMD D2 TNP03-related (LGMD D2), LGMD D3 HNRNPDL-related (LGMD D3), LGMD D4 calpain3-related (LGMD D4) and LGMD D5 collagen6-related (LGMD D5).

The different types of limb girdle muscular dystrophies that have been cited are in accordance to the new nomenclature agreed at the 229^{th} ENMC International workshop (Neuromuscular Disorders, 2018, 28, 702-710). This classification follows the formula: LGMD, inheritance (R for recessive and D for dominant), order of discovery (number) and affected protein. In a preferred embodiment, the limb girdle muscular dystrophy is limb-girdle muscular dystrophy R1 calpain 3-related, also referred to as LGMDR1 or calpainopathy.

The terms "treating" and "treatment", as used herein, means reversing, alleviating, inhibiting the progress of, the disease or condition to which such term applies, or one or more symptoms of such disease or condition, such as lowering the viral load in a patient with respect to pretreatment levels.

The term "subject" refers to a mammal, e.g., a human.

In particular, the compounds for use according to the invention are administered as a pharmaceutical composition, which comprises the corresponding active compound of formula (I) and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are known by the skilled person. The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a nontoxic but sufficient amount of the corresponding compound to provide the desired effect. The amount that is "effective" will vary from subject to subject, depending on the age and general condition of the individual, the particular compound administered, and the like. However, an appropriate amount in any individual case may be determined by one of ordinary skill in the art using routine experimentation.

The compounds for use according to the invention may be administered by any appropriate route (via), such as oral (e.g., oral, sublingual, etc.) or parenteral (e.g., subcutaneous, intramuscular, intravenous, etc.).

Examples of pharmaceutical compositions include any solid (tablets, pills, capsules, granules etc.) or liquid (solutions, suspensions or emulsions) composition for oral or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the European and US Pharmacopoeias and similar reference texts.

The compounds for use according to the invention will typically be administered once or more times a day, for example 1, 2, 3 or 4 times daily, with typical total daily doses depending on the particular compound and severity of the disease, and may be easily determined by the skilled practitioner.

By way of example, typical total daily doses the compounds of the invention are in the range of from 0.1 to 1000 mg/day, preferably from 1 to 600 mg/ day, even more preferably from 1 to 100 mg/day.

The compounds and compositions for use according to the invention may be administered as the sole active ingredient, or in combination with other active ingredients to provide a combination therapy. The other active ingredients may form part of the same composition, or be provided as a separate composition for administration at the same time or at different time.

In a particular embodiment, the compounds of the invention are used in combination with one or more compounds useful in the treatment of limb girdle muscular dystrophy, more preferably calpainopathy or limb-girdle muscular dystrophy R1 calpain 3-related (LGMDR1).

The term "combination" refers to a product comprising one or more of the defined compounds, either in a single composition or in several compositions (or units), in which case the corresponding compounds are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the compounds or compositions (or units) are administered at the same time.

Sequential administration means that the compounds or compositions (or units) are administered at different time points in a chronologically staggered manner.

Separate administration means that the compounds or compositions (or units) are administered at different time points independently of each other.

In particular, the combinations for use according to the invention are administered as pharmaceutical compositions, which comprise the corresponding (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

The following examples are intended to further illustrate the invention. They should not be interpreted as a limitation of the scope of the invention as defined in the claims.

### Examples

### Materials and Methods

### Muscle biopsy samples and primary cell culture

All participants gave informed consent for sample collection (Table 1 below). Proximal muscle samples (quadriceps, deltoid, biceps and triceps) were obtained from healthy controls and LGMDR1 patients, in whom the diagnosis was genetically confirmed by identification of the two mutations in the calpain 3 gene. One fraction of the biopsies was used to obtain protein, the other to obtain myoblasts.

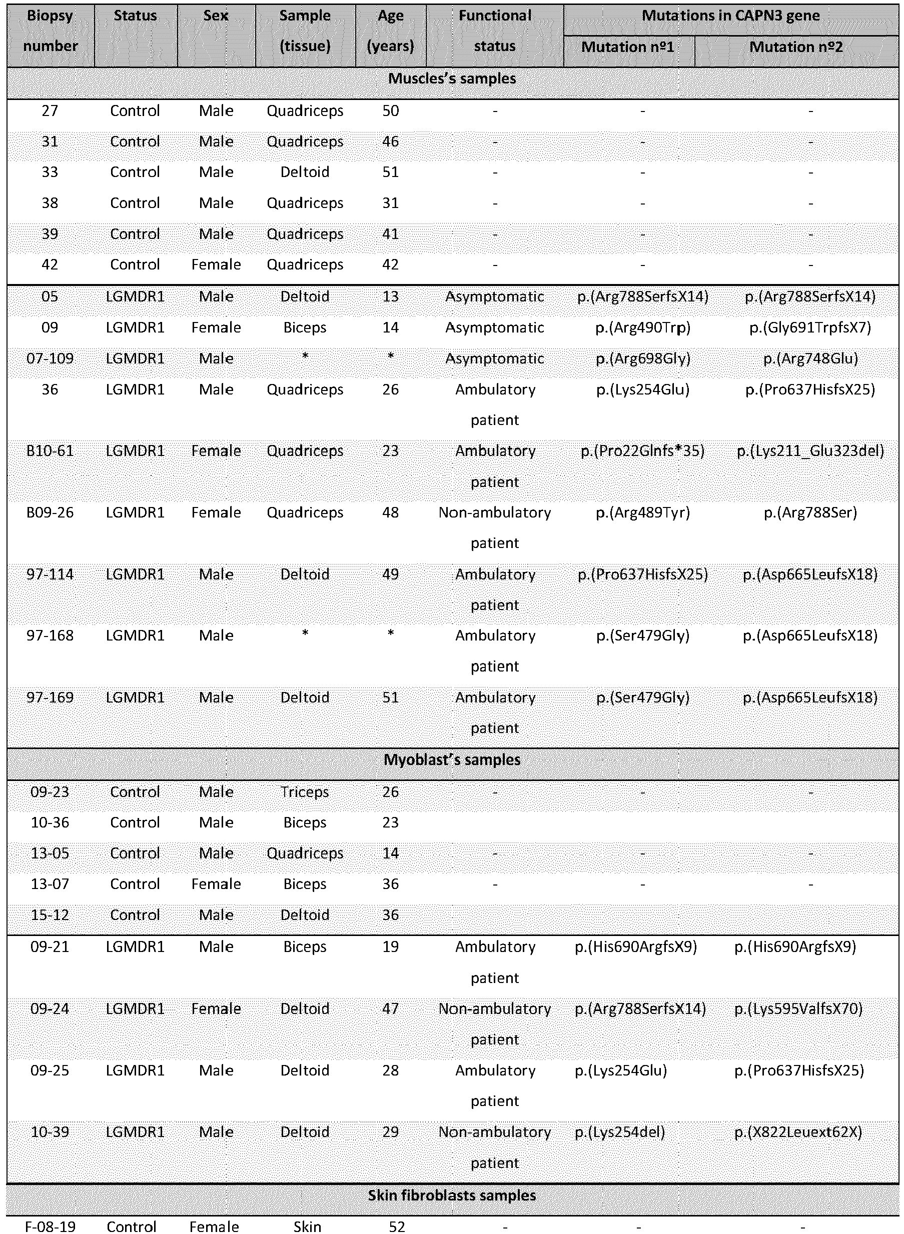

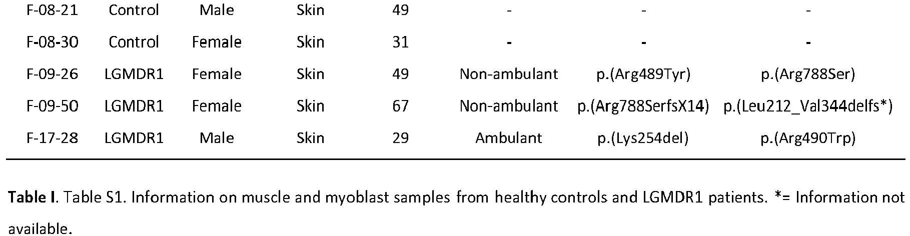

To obtain primary myoblasts, muscle biopsies were processed and cultured in monolayer according to the method described by Askanas (Neurology, 1975, 25, 58-67). To isolate the pure population of myoblasts and CD56-negative cells (CD56-), the protocol described previously (Jaka et al., Expert Reviews in Molecular Medicine, 2017, 19, e2) was used, using immunomagnetic separation based on the early presence of the surface marker CD56 (Miltenyi Biotec, 130-050-401).

Myoblasts were cultured in proliferation medium (DMEM, M-199, Insulin, Glutamine and penicillin-streptomycin) with 10% FBS and growth factors at 37 °C in 5% CO₂ air incubator. When myoblasts reached confluence, the FBS concentration was reduced to 2% and growth factors were removed from the medium, promoting fusion and production of myotubes.

### Skin biopsies and fibroblast culture

Skin fibroblasts from healthy controls and from LGMDR1 patients were provided by Dr. Ana Aiastui (Biodonostia Health Research Institute, Cell Culture and Histology Platform). Cells were cultured in Dulbecco's modified Eagle's medium (DMEM, Gibco, 41966-029) supplemented with 10% FBS (Gibco, 10270-106) and 1% penicillin-streptomycin (Gibco, 15140-122) at 37 °C in a 5% CO₂ air incubator. Human fibroblasts were cultured at a density of 4x10⁴ cells/ml.

### FRZB gene silencing

FRZB gene silencing in primary human myotubes has been performed using interfering RNA as previously described (Jaka et al., Expert Reviews in Molecular Medicine, 2017, 19, e2). The siRNA for FRZB gene silencing (s5369) and the siRNA used as negative control (AM4611) were obtained from Life Technologies. Cells plated at 24 000 cells/cm² were transfected with the siRNA at a concentration of 5 nM using RiboCellin transfection reagent (Eurobio) following the manufacturer's instructions. After 8 days of differentiation, human primary myotubes were incubated with the corresponding siRNA and the transfection agent. Finally, the RNA obtained from these cultures was analysed 48 h post-transfection by quantitative real-time PCR. Likewise, proteins from these cultures were analysed 72 h post-transfection.

### Administration of compounds

VP0.7 was provided by Dr. Ana Martinez (from The Center for Biological Research, CSIC). It was administered at 1.2 µM, whereas LiCI (Sigma-Aldrich, L7026) was used for comparative purposes at a final concentration of 10 mM. VP0.7 and LiCI were administered at the above referred concentrations to myotubes at eight days of differentiation, and VP0.7 also to skin fibroblasts and to CD56- cells. Negative controls, in the case of VP0.7, were performed with DMSO (Fisher Scientific, BP-231). At 48 hours after drug administration, RNA and proteins were extracted.

### RNA extraction from myoblast/myotubes and muscle biopsies

RNA extraction from primary myoblast/myotubes was performed with an RNeasy Mini Kit (Qiagen). In the case of the muscle biopsies, which were snap frozen and stored at -80°C until use, total RNA was obtained using an RNA-Plus Kit (QBiogene).

### Quantitative real-time PCR

The isolated RNA was reverse-transcribed to first-strand complementary DNA (cDNA) in a final volume of 50 µl using a High Capacity cDNA Reverse Transcription Kit (Applied Biosystems), according to the manufacturer's instructions. The expression levels of CAPN3, FOS, ITGB1BP2, ANOS1 and GAPDH genes in the samples were assessed by real-time quantitative PCR with TaqMan probes (Table II below) using the 'CFX384 Real-Time PCR Detection System' (Bio-Rad).

**Table II. TaqMan probes.**

| **Gene and exon to which the probe binds** | **Probe's code** |
|---|---|
| *CAPN3* Ex1-2 | Hs00181057_m1 |
| *FOS* Ex1-2 | Hs99999140_m1 |
| *ITGB1BP2* Ex4-5 | Hs00183746_m1 |
| *ANOS1* Ex2-3 | Hs01085107_m1 |
| *GAPDH* Ex2 | Hs99999905_m1 |

### Protein extraction and Western blot analysis

Protein collection from muscle samples and cell cultures, as well as Western blot procedure, were performed as previously described (Jaka et al., Expert Reviews in Molecular Medicine, 2017, 19, e2). The antibodies used are listed in Table III below.

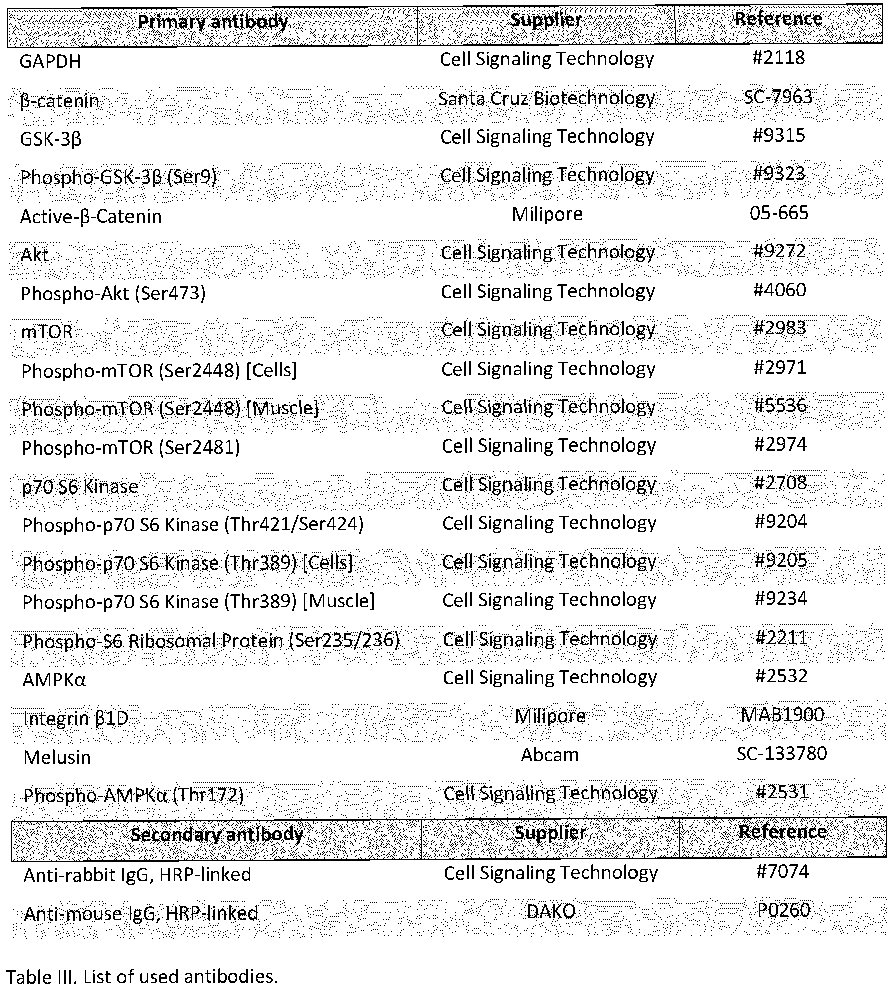

### Example 1. Alterations in Wnt and mTOR pathways involved in LGMDR1

### 1. Alteration of the Wnt/β-catenin pathway in the muscle of LGMDR1 patients

Previous studies had described the overexpression of FRZB, a Wnt1, 5a, 8 and 9a antagonist, in the muscle of LGMDR1 patients (Jaka et al., Expert Reviews in Molecular Medicine, 2017, 19, e2). Due to the excessive inhibition of this pathway, the expression of some proteins participating in the Wnt pathway has been analysed.

Namely, the levels of GSK3β and total β-catenin were tested, although no significant differences were observed between patients and controls. However, the active form of β-catenin, as well as the phosphorilation form of GSK3 β (Ser9), showed a drastic decrease of these proteins in patients (Figure 1A). These results have allowed establishing that in the muscle of LGMDR1 patients there is a reduction of active β-catenin, which confirms that the Wnt pathway is altered.

### 2. Reduction in the activation of the mTOR pathway in the muscle of LGMDR1 patients

The expression of mTOR as well as its phosphorylated form in Ser2448 were severely reduced in muscle of LGMDR1 patients (p=0.00159; p=0.00159 respectively). The decrease of both forms can be observed in pseudoasymptomatic and symptomatic patients (Figure 1B).

Subsequently, the expression of kinases downstream of this pathway was analysed. p70S6K is a kinase participating in the mTOR pathway and that is activated by sequential phosphorylations (Pullen et al., FEBS Letters, 1997, 410(1), 78-82; Fenton et al., The International Journal of Biochemistry & Cell Biology, 2011, 43(1), 47-59). Our results show that the levels of its phosphorylation form at the Thr421/Thr424 residues (Figure 1C) showed a greatly decreased. Downstream, the kinase p70S6K catalyzes the phosphorylation of ribosomal protein S6 (RPS6), whose phosphorylation correlates with high levels of protein translation and synthesis (Nielsen et al. The Journal of Biological Chemistry, 1982, 257(20), 12316-12321; Fenton et al., The International Journal of Biochemistry & Cell Biology, 2011, 43(1), 47-59). When analysing the phosphorylation of RPS6 (Ser235/Ser236), a statistically significant decrease was found in the muscle of LGMDR1 patients when analyzed together (p = 0.03, data not shown, and Figure 1C).

On the other hand, being known that AMPK kinase is an inhibitor of the mTOR pathway (Du et al., Biochemical and Biophysical Research Communications, 2008, 368(2), 402-407; Hwang et al., British Journal of Pharmacology, 2013, 169(1), 69-81), it was analysed in the muscle of patients and an increase in the amount of this kinase was observed in the group of patients (p = 0.038) (Figure 1C).

These results point out that alterations in the mTOR signalling pathway are also present in muscle from LGMDR1 patients, since severe decrease in mTOR expression, its phosphorylation, as well as a reduction in the activation of downstream proteins of the pathway have been observed, which leads to dysregulation of the growth of muscle fibre.

### Example 2. In vitro treatment with VP0.7

VP0.7 was administered according to the procedure mentioned above in different cell types: primary myotubes, skin fibroblasts and CD56-cells. Li has also been used for comparative purposes.

The effect on the response for the administered drugs was different depending on the cell type as shown below.

### 2.1. Activation of the Wnt pathway in human myotubes and recovery of deregulated gene expression in LGMDR1 patients

We proceeded to analyse the expression of the GSK3β protein, as well as its downstream effect on the Wnt pathway in myotubes from controls and patients after drug administration. The activation of the Wnt pathway was confirmed by effective inhibition of GSK3β kinase, since an increase in total β-catenin expression (Figure 2A) as well as active β-catenin (Figure 2B) was observed with VP0.7. As a control assay, lithium was administered and an increase in active β-catenin was verified (Figure 2C).

On the other hand, in previous studies we had already described that activating the Wnt pathway through the silencing of the FRZB gene or with treatment with lithium, the expression of some altered genes in the muscle of patients was recovered (Jaka et al., Expert Reviews in Molecular Medicine, 2017, 19, e2; Casas -Fraile et al., Orphanet Journal of Rare Diseases, 2020, 15(1), 119). Thus, their expression was analysed after administering VP0.7. The results showed a correlation with the effect generated by the FRZB gene silencing, that is, an increase in the expression of the CAPN3, FOS and ANOS1, and an increase in ITGB1BP2 was also observed (Figure 2D).

Likewise, the expression of some of the structural proteins that are part of the costamere, whose expression is altered in LGMDR1 patients, was analysed. After drug administration, an increase in integrin β1D and melusin was also observed (Figure 2E).

### 2.2. Activation of the Akt/mTOR pathway in myotubes from LGMDR1 patients

After administration of VP0.7 in the patient's myotubes, no significant changes were observed in total mTOR, but there was a significant increase in phosphorylation of mTOR in two of its residues (Ser2448 and Ser2481) (Figure 3A).

Furthermore, downstream of the mTOR pathway, the p70S6K and RPS6 proteins were analysed. After administration of VP0.7, the expression of total p70S6K, its phosphorylation (Thr421/Ser424 and Thr389), as well as the levels of phosphorylation of the RPS6 protein (Ser235/Ser236) increased in the patient's sample. In the control, the effect observed after the treatments is very slight (Figure 3B).

These results have shown that the administration of a compound of formula (I) as the ones described above, and particularly VP0.7, in myotubes increases the amount of β-catenin which in turn leads to an activation of the Wnt pathway, as well as also activates the mTOR pathway.

For comparative purposes, LiCI was also administered in control and patient's myotubes and the phosphorylation of p70S6K and RPS6 proteins were also tested. The results are depicted in Figure 4.

It can be observed that the administration of lithium did not provide any increase in the proteins involved in the mTOR pathway, nor in any of their phosphorylation forms, thus confirming that this compound is not effective for regulating one of the most important signalling pathways involved in LGMDR1 in spite of having been described its use in the treatment of this disease in the prior art.

The comparative results between lithium and VP0.7 are also expressed in Tables IV and V, wherein the increase in the expression of p70S6K, its phosphorylation (Thr421/Ser424 and Thr389), and in the levels of phosphorylation of the RPS6 protein upon administration of VP0.7 in patient's sample is observed when compared to the administration of lithium.

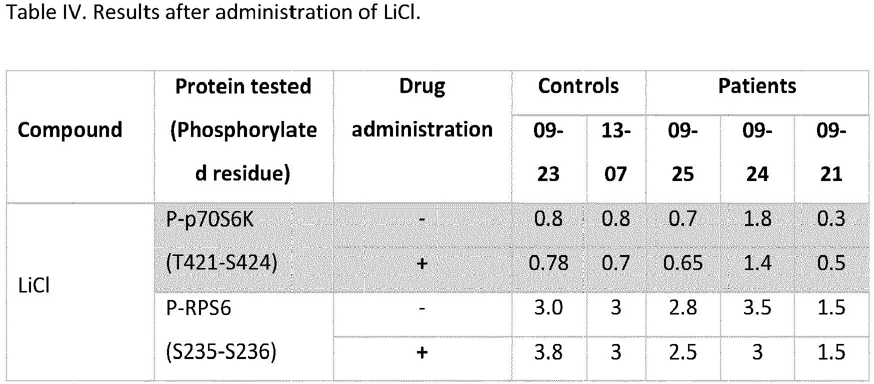

**Table V. Results after administration of VP0.7.**

| **Compound** | **Protein tested (Phosphorylated residue)** | **Control 13-05** | **Patient 09-25** |
|---|---|---|---|
| VP0.7 | P-p70S6K (T421-S424) | 1 | 1.6 |
| | P-p70S6K (T389) | 0.94 | 1.6 |
| | P-RPS6 (S235-S236) | 1.1 | 2.8 |

*The values shown in the table correspond to the protein expression value relative to that of the same sample without treatment.*

Thus, the fact that compounds of formula (I), such as VP0.7, recover both, the Wnt and mTOR signalling pathways in LGMDR1 patients is of great importance for the treatment of this disease as they can be more effective than other known compounds to recover the altered homeostasis of the muscle fibre.

### 2.3. Absence of activation of the Wnt pathway in fibroblasts and CD56- after treatment with VP0.7

In order to establish whether the effects previously observed after drug administration in myogenic cells (myoblasts differentiated to myotubes) occurred similarly in other cell types, we proceeded to study skin fibroblasts and CD56- cells obtained from patients' muscle.

### Fibroblasts

Treatment with VP0.7 in fibroblasts did not show an increase in the phosphorylation of GSK3β (Ser9) (Figure 5A), nor in β-catenin or its active form, indicating that the Wnt pathway is not activated in this cell type by means of VP0.7. Furthermore, the effect that the inhibition of GSK3β could cause in the mTOR pathway was also tested, observing only a very slight increase in the phosphorylation of RPS6 (Figure 5B).

### CD56- cells

After treatment with VP0.7 in CD56-cells, no significant alteration was observed in the amount of total β-catenin or in its active form (Figure 6A). Finally, the effect of this inhibition on the regulation of the mTOR pathway was also tested, but no differences were observed in mTOR or in the phosphorylations of p70S6K and RPS6 (Figure 6B).

Thus, the administration of VP0.7 did not activate the Wnt or mTOR pathways in fibroblasts or CD56-cells. The absence of effect after treatment with VP0.7 in different cell types is of great interest, since it indicates that the treatment is not affecting other tissues avoiding undesired adverse effects.

## Claims

1. A compound of formula (I): 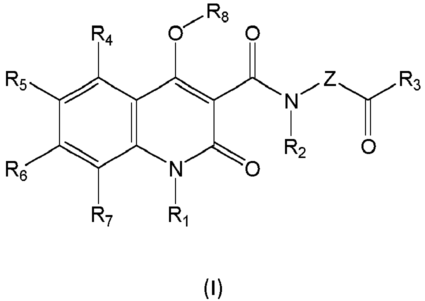 wherein:
R₁ is selected from H, an optionally substituted C₁-C₁₂ alkyl group and an optionally substituted C₂-C₅ alkenyl group;
R₂ is H or a C₁-C₆ alkyl group;
R₃ is an optionally substituted C₁-C₂₀ alkyl group;
R₄-R₇ are independently selected from H, halogen, an optionally substituted C₁-C₆ alkyl group and -OR₉, wherein R₉ is hydrogen or an optionally substituted C₁-C₆ alkyl;
R₈ is selected from H and an optionally C₁-C₆ alkyl group;
Z is selected from -NR₁₀- and an optionally substituted phenylene; wherein R₁₀ is selected from H and an optonally substituted C₁-C₄ alkyl;
or a pharmaceutically acceptable salt or solvate thereof,
for its use in the treatment of limb girdle muscular dystrophy.

2. Compound for use according to claim 1, wherein R₁ is H, a linear or branched C₁-C₆ alkyl group optionally substituted with an aryl or cycloalkyl group, or a non-substituted C₂-C₅ alkenyl group.

3. Compound for use according to claim 2, wherein R₁ is a non-substituted linear or branched C₁-C₆ group.

4. Compound for use according to any one of the preceding claims, wherein R₂ is H or a non-substituted C₁-C₃ alkyl group.

5. Compound for use according to claim 4, wherein R₂ is H.

6. Compound for use according to any one of the preceding claims, wherein R₃ is a non-substituted linear C₄-C₁₅ alkyl group.

7. Compound for use according to any one of the preceding claims, wherein R₄-R₇ are independently selected from H and halogen.

8. Compound for use according to any one of the preceding claims, wherein Z is selected from -NH- and a non-substituted phenylene.

9. Compound for use according to any one of the preceding claims, wherein:
- R₁ is a non-substituted C₁-C₆ alkyl group;
- R₂ is H;
- R₃ is a non-substituted linear C₄-C₁₅ alkyl group;
- R₄-R₇ are independently selected from H and halogen;
- Z is selected from -NH- and non-substituted phenylene.

10. Compound for use according to any one of the preceding claims selected from: 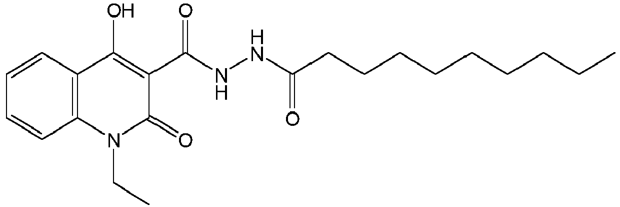 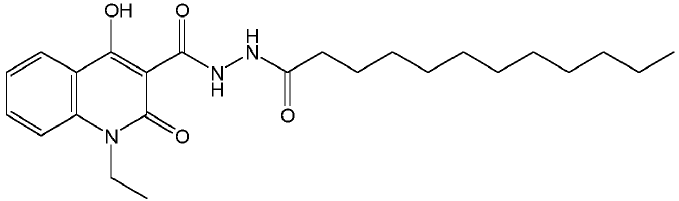 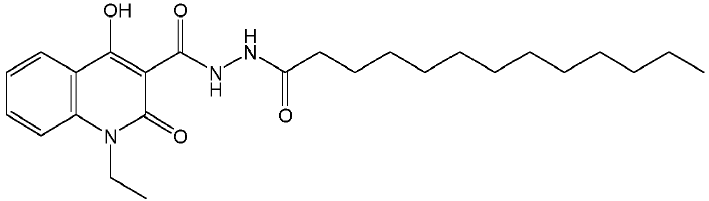 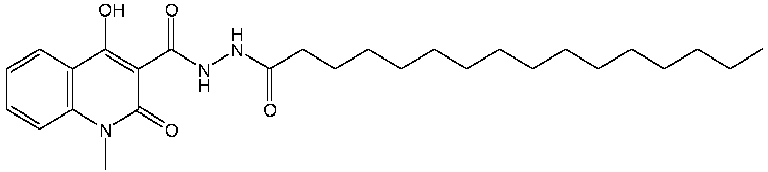 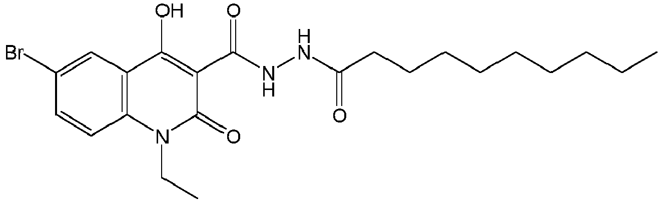 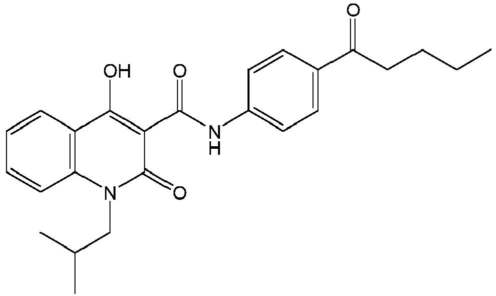 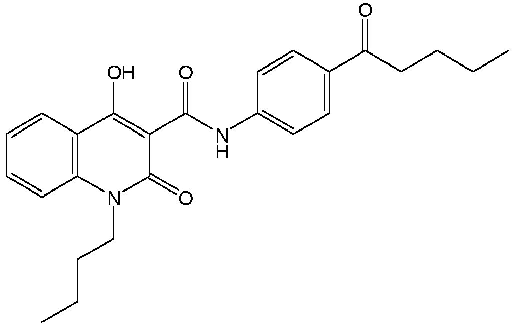 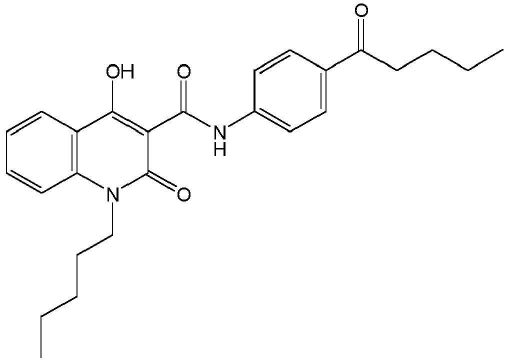 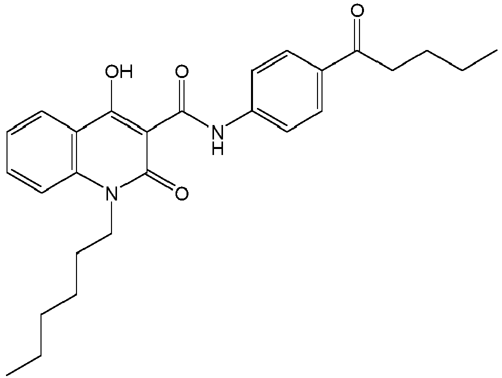 and their salts or solvates.

11. Compound for use according to any one of the preceding claims, which is: 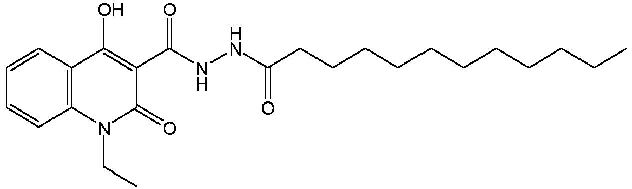 or a salt or solvate thereof.

12. Compound for use according to any one of the preceding claims, wherein the limb girdle muscular dystrophy is a recessive limb girdle muscular dystrophy selected from LGMD R1 calpain3-related (LGMDR1 or calpainopathy), LGMD R2 dysferlin-related (LGMDR2), LGMD R3 α-sarcoglycan-related (LGMDR3), LGMD R4 β-sarcoglycan-related (LGMDR4), LGMD R5 γ -sarcoglycan-related (LGMDR5), LGMD R6 δ-sarcoglycan-related (LGMDR6), LGMD R7 telethonin-related (LGMDR7), LGMD R8 TRIM 32-related (LGMDR8), LGMD R9 FKRP-related (LGMDR9), LGMD R10 titin-related (LGMDR10), LGMD R11 POMT1-related (LGMDR11), LGMD R12 anoctamin5-related (LGMDR12), LGMD R13 Fukutin-related (LGMDR13), LGMD R14 POMT2-related (LGMDR14), LGMD R15 POMGnT1-related (LGMDR15), LGMD R16 α-dystroglycan-related (LGMDR16), LGMD R17plectin-related (LGMDR17), LGMD R18TRAPPC11-related (LGMDR18), LGMD R19GMPPB-related (LGMDR19), LGMD R20ISPD-related (LGMDR20), LGMD R21POGLUT1-related (LGMDR21), LGMD R22collagen6-related (LGMDR22), LGMD R23laminin α2-related (LGMDR23), and LGMD R24POMGNT2-related (LGMDR24); or a dominant limb girdle muscular dystrophy selected from LGMD D1 DNAJB6-related (LGMD D1), LGMD D2 TNP03-related (LGMD D2), LGMD D3 HNRNPDL-related (LGMD D3), LGMD D4 calpain3-related (LGMD D4) and LGMD D5 collagen6-related (LGMD D5).

13. Compound for use according to claim 13, wherein the limb girdle muscular dystrophy is limb-girdle muscular dystrophy R1 calpain 3-related.

14. Compound for use according to any one of the preceding claims, which is used in combination with one or more active ingredients to provide a combination therapy, wherein the other active ingredients may form part of the same composition, or be provided as a separate composition for administration at the same time or at different time.
